(19) Europäisches Patentamt
European Patent Office
Office européen des brevets

(11) Numéro de publication: **0 079 842**
**B1**

# (12) FASCICULE DE BREVET EUROPEEN

(45) Date de publication du fascicule du brevet: **14.08.85**

(51) Int. Cl.⁴: **C 08 F 220/54**, B 01 J 39/20, B 01 J 39/06

(21) Numéro de dépôt: **82402110.9**

(22) Date de dépôt: **19.11.82**

## (54) Nouveaux copolymères acryliques à base de N-acrylylpolyméthylèneimines ou de N-acrylyldialcoylamides, de N,N'-acrylyldiaminoalcanes et de N-acrylylaminoacides (ou esters), leur préparation et leur emploi comme échangeurs cationiques.

(30) Priorité: **19.11.81 GB 8134861**

(43) Date de publication de la demande:
**25.05.83 Bulletin 83/21**

(45) Mention de la délivrance du brevet:
**14.08.85 Bulletin 85/33**

(84) Etats contractants désignés:
**AT BE CH DE FR GB IT LI LU NL SE**

(56) Documents cités:
**DE - B - 1 212 729**
**FR - A - 1 506 798**
**FR - A - 2 197 897**

(73) Titulaire: **Société d'Expansion Scientifique EXPANSIA Société Anonyme, 51/53 rue du Docteur Blanche, F-75016 Paris (FR)**

(72) Inventeur: **Aspisi, Christian, Les Grands Vallons Chemin du Breuil, F-13150 Boulbon (FR)**
Inventeur: **Calas, Bernard, 9, Rue de la Traversière, F-34980 St. Gely du Fesc (FR)**
Inventeur: **Daunis, Jacques, 38 bld. Ernest Renan, F-34000 Montpellier (FR)**
Inventeur: **Follet, Michel, 1 Ave. de la Libération, F-30390 Aramon (FR)**
Inventeur: **Jacquier, Robert, 445 Av. Valéry Larbaud, F-34000 Montpellier (FR)**
Inventeur: **Parello, Joseph, 26 rue Haguenot, F-34000 Montpellier (FR)**

(74) Mandataire: **Lachassagne, Jacques, Boite Postale 07, F-78430 Louveciennes (FR)**

EP 0 079 842 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention a pour objet de nouveaux copolymères acryliques statistiques réticulés, utilisables dans une très large gamme de solvants (eau, chlorure de méthylène, chloroforme, méthanol, éthanol, diméthylformamide, pyridine, etc.), leurs procédés de préparation et leur emploi dans la chromatographie d'échange d'ions.

La technique de la chromatographie d'échange d'ions consiste en l'emploi d'un support insoluble (matrice) sur lequel sont fixés des groupes ionisables associés à des ions de signes contraires (contre-ions).

Ces derniers peuvent être échangés avec d'autres ions de même signe, contenus dans une phase mobile que l'on met en contact avec la matrice. Selon le caractère acide ou basique des groupes fixés, on parlera d'une résine échangeuse de cations ou d'anions.

Cette technique est applicable à la séparation des différents constituants d'un mélange en solution; les molécules non ionisées seront éluées rapidement, les molécules ionisées seront plus ou moins retenues en raison de l'échange d'ions intervenant avec la matrice.

La présente invention concerne de nouveaux copolymères utilsables notamment comme résines échangeuses de cations.

Il existe actuellement un certain nombre de résines acryliques ou méthacryliques, possédant des groupes acides carboxyliques, qui sont utilisées comme support en chromatographie d'échange d'ions (échangeurs de cations).

Elles sont généralement obtenues à partir de polymères non fonctionnels, préparées par copolymérisation radicalaire de dérivés de l'acide acrylique ou méthacrylique avec un agent de réticulation qui peut être soit le méthylène bis acrylamide (N,N'-diacryloyldiaminométhane, abréviation MBA), soit le divinylbenzène.

Dans la majorité des cas, les fonctions acides sont alors introduites dans la matrice par des modifications chimiques intervenant après la polymérisation. Ces réactions sont difficiles à contrôler et peuvent conduire à un manque d'homogénéité au niveau de la fonctionnalisation (hydrolyse de la maille ou de l'agent de réticulation).

Ainsi, le copolymère d'acrylamide et de méthylène bis acrylamide (connu sous la marque commerciale Biogel P appartenant à Biorad Laboratories) donne, après hydrolyse partielle par la soude, le support connu sous la marque commerciale Biogel 70 (Inman et Dintzis, «Biochemistry», 1969, *8*, 4074).

Il ressort de la structure chimique de ces résines (figure ci-dessous) que la fonction carboxylique est adjacente à la chaîne du polymère; par conséquent, des contraintes stériques gêneront l'interaction entre des molécules volumineuses et le site acide.

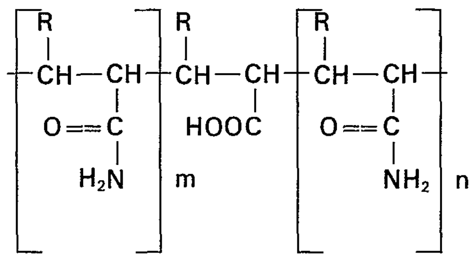

R = H ou $-CH_3$

La même critique peut être formulée à l'encontre d'autres résines comme celles connues sous la marque commerciale Trisacryl appartenant à Pharmindustrie (brevets français Nos 2378808 et 2482112), qui, elles, sont obtenues par la copolymérisation avec un monomère fonctionnalisé.

En outre, certains gels de polyacrylamide (Biogel, Duolite) sont chimiquement instables en milieu basique du fait de l'hydrolyse des groupes amides en groupes carboxyliques, ce qui limite leur domaine d'emploi (Inman et Dintzis, déjà cité).

Il faut noter également les limitations introduites par le manque de stabilité mécanique des polyacrylamides à fonction amide primaire (Scouten, «Affinity Chromatography: bioselective absorption on inert matrices», Wiley, 1981).

On peut, enfin, mentionner une résine décrite par Sheppard et coll. («J.C.S. Perkin Trans I», 1981, pp. 529 et 538), résultant de la copolymérisation du N-acrylyldiméthylamide, du N,N'-diacrylyldiamino-1, 2-éthane et de l'ester méthylique de la N-acrylylsarcosine. Cependant, ce support n'a jamais été hydrolysé en copolymère de N-acrylyldiméthylamide, N,N'-diacrylyldiamino-1, 2 éthane et N-acrylylsarcosine, pour être utilisé en tant qu'échangeur de cation. Si l'on effectue cette transformation et teste la résine acide ainsi obtenue, en chromatographie d'échange d'ions, on constate des variations de volume très importantes en fonction de la force ionique de l'éluant. Ainsi, en travaillant avec des solutions de chlorure de sodium de concentration progressive comprise entre 0 et 0,5 M, on peut noter une contraction de volume supérieure à 50% du support de Sheppard hydrolysé; cela induit un volume mort important et, par là même, une forte incertitude sur la valeur du gradient d'élution; d'où l'utilisation délicate de ce type de support en chromatographie d'échange de cations.

Afin de proposer un nouveau type de copolymère ne présentant pas les inconvénients de ceux déjà connus, il a été mis au point, conformément à la présente invention, un nouveau type polyvalent de copolymères statistiques réticulés, par copolymérisation de trois monomères distincts définis comme suit:

I. Un monomère défini comme étant la maille du support et qui consiste en une N-acrylylpolyméthylèneimine de formule:

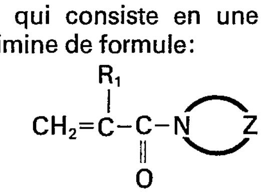

(1)

avec $R_1 = H$ ou $-CH_3$

$Z = -(CH_2)_{n1}-$ avec n = 4, 5 ou 6

ou

$-(CH_2)_2-X-(CH_2)_2-$

avec X = O ou $-N(CH_3)-$

ou un N-acrylyldialcoylamide, de formule:

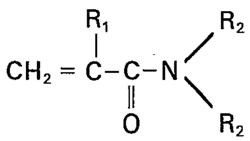
(2)

avec $R_1$ ayant la signification précédente et $R_2 = -CH_3$ ou $-C_2H_5$.

II. Un monomère défini comme étant l'agent de réticulation et qui consiste en un N,N'-bis-acrylyldiaminoalcane de formule:

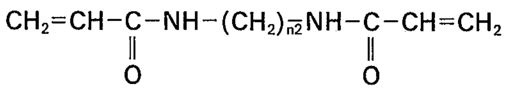
(3)

avec $n_2$ = 1 ou 2.

III. Un monomère défini comme étant l'agent de fonctionnalisation et qui consiste en un N-acrylylaminoacide ou ester, de formule:

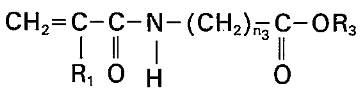
(4)

avec
$R_1 = H$ ou $-CH_3$
$R_3 = H$ ou $-CH_3$
$n_3$ = 1, 2, 3 ou 5

ou un N-acrylylaminoacide (ou ester) asymétrique de série L, de formule:

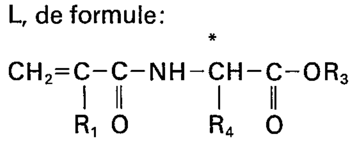
(5)

avec
$R_1 = H$ ou $-CH_3$
$R_3$ ayant la signification précédente
$R_4 = -CH_3$
$-CH(CH_3)_2$
$-CH_2-CH(CH_3)_2$

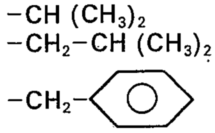

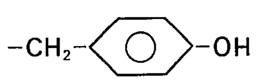

$-CH_2-CH_2-S-CH_3$
$-(CH_2)_4-NH_2$

ou encore la N-acrylyl (L) proline, ou son ester méthylique, de formule:

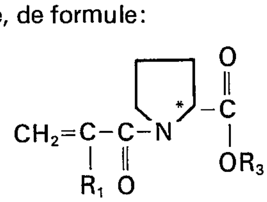
(6)

avec
$R_1 = H$ ou $-CH_3$
$R_3$ ayant la signification précédente.

L'utilisation comme monomère servant de maille, de N-acrylylpolyméthylèneimine 1 ou de N-acrylyldialcoylamide 2, avec des fonctions amides disubstituées, permet de supprimer totalement l'intervention des liaisons hydrogène. Aussi, les résines préparées ont un fort taux de gonflement dans une large gamme de solvants organiques (protiques et aprotiques) et dans des tampons aqueux de divers pH.

La fonctionnalisation des supports, réalisée par l'emploi de N-acrylylaminoacides (ou esters) 4, 5 ou 6, rend possible la modification de la nature et de la longueur du bras séparant la fonction acide ou ester du squelette polymérique.

Certains monomères intervenant dans la maille sont connus. Ils ont été décrits, avec leurs procédés de préparation, dans diverses publications parmi lesquelles:

Composés 1: $R_1 = H$, $Z = -(CH_2)_4-$, $-(CH_2)_5-$ Z $= -(CH_2)_2-O-(CH_2)_2-$

Parrod et coll., «J. Polymer. Sci.», 1958, *29*, 111.

Composés 2: $R_1 = H$, $R_2 = -cH_3$

Ratchford et coll., «J. Amer. Chem. Soc.», 1947, *69*, 1911.

$R_1 = H$, $R_2 = -C_2H_5$

Brevet américain N° 2683741.

Composés 3:

Les agents de formule 3 sont connus:

$n_2$ = 1 (commercial)

$n_2$ = 2

Sheppard et coll., «J. Chem. Soc. Perkin I», 1981, 529.

Enfin, certains agents de fonctionnalisation de formule 4, 5 ou 6 ont été décrits dans la littérature.

Composés 4:

$R_1 = R_3 = H$, $n_3$ = 1, 2, 3 ou 5

Brown et coll., «C.R. Acad. Sci.», 1978, *287*c, 125.

$R_1 = CH_3$, $R_3 = H$, $n_3$ = 1, 2, 3 ou 5

Batz et Koldehoff, «Makromol. Chem.», 1976, *177*, 683.

Winston et Kirchner, «Macromolécules», 1978, *11*, 89.

Loupek et coll., «Bioch. Biophys. Act.», 1977, *481*, 289.

Composés 5:

$R_1 = H$, $R_3 = CH_3$

$R_4 = -CH_3$, 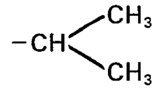 , 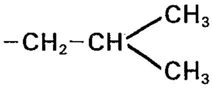

$-CH_2-CH_2-S-CH_3$ ou 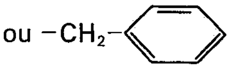

Sakota, «Koine Nippon Zagakin Zashi», 1967, *88* (10), 1087.

La copolymérisation de ces deux catégories de monomères I d'une part, III, d'autre part, utilisés respectivement en tant que maille et agent de fonctionnalisation, avec un agent de réticulation de type II, permet d'obtenir des résines ayant des comportements physiques compatibles avec les

chromatographies d'échange d'ions, en particulier en ce qui concerne le débit d'élution. Ainsi, les contractions de volume en fonction de la force ionique de l'éluant aqueux n'ont jamais été supérieures à 15%.

Les conditions de polymérisation étant parfaitement contrôlées et les rendements élevés, il est possible de faire varier dans une large gamme le taux de fonctionnalisation. Pour cela, il suffit de modifier la quantité relative de N-acrylylaminoacide (ou ester) III, par rapport aux deux autre monomères.

Les copolymères selon l'invention sont des copolymères statistiques réticulés tridimensionnels, contenant sous forme copolymérisée:

a) de 30 à 90% en poids d'un monomère correspondant à une N-acrylylpolyméthylèneimine de formule 1 ou à un N-acrylyldialcoylamide de formule 2;

b) de 2 à 50% en poids d'un monomère correspondant à un N,N'-diacrylyldiaminoalcane de formule 3;

c) de 2 à 65% en poids d'un monomère correspondant à un acrylylaminoacide ou ester, racémique de formule 4 ou optiquement actif de formule 5 ou 6.

Les copolymères selon l'invention contiennent de préférence:

– de 70 à 90% de monomère I (définissant la maille) correspondant aux formules 1 et 2;

– de 3 à 10% de monomère II (agent de réticulation) correspondant à la formule 3;

– de 10 à 20% de monomère III (agent de fonctionnalisation) correspondant aux formules 4, 5 et 6.

Selon l'invention on a pu préparer des supports contenant de 0,2 à 10 mEq en groupe carboxylique par gramme de support sec (directement ou après saponification). Il a été noté, d'autre part, qu'aucune hydrolyse de la fonction ester n'intervient lors de la polymérisation.

Les copolymères sous forme acide sont chimiquement stables en milieu nettement acide ou basique. Des traitements de plusieurs jours, par des solutions d'acide chlorhydrique ou de soude 0,5 et 1 M, à température ambiante, ont montré qu'aucune hydrolyse, même partielle, n'intervenait.

Cela est lié à la présence, sur le monomère formant la maille, d'un amide secondaire relativement encombré, beaucoup plus stable que les amides primaires généralement utilisés. Cela permet l'emploi de nos supports dans un domaine de pH très étendu (1 à 14). Les résines obtenues présentent des pK variant de 4,5 à 5,0.

Ces copolymères sont stables thermiquement; en effet, par chauffage à 90° C pendant 12 h, sous vide de 0,1 mm de mercure, on ne constate aucune modification.

Enfin, ces supports présentent une excellente stabilité mécanique. Après agitation mécanique pendant plusieurs jours, on ne constate pas de formation de fines (aucune turbidité dans le liquide surnageant).

Les copolymères réticulés selon l'invention peuvent être préparés, selon des procédés connus, par polymérisation radicalaire des monomères I, II et III.

La copolymérisation peut en particulier être effectuée soit en bloc, dans un mélange alcool éthylique/eau ou diméthylformamide/eau en milieu tamponné (pH = 6,0), soit en suspension dans les systèmes eau/paraffine ou alcool polyvinylique/eau. L'amorçage est réalisé par les initiateurs habituellement utilisés en polymérisation radicalaire. Comme tels, on peut citer:

– les couples comme N,N,N',N'-tétraméthyléthylènediamine (TMEDA) + persulfate alcalin,

– le persulfate d'ammonium,

– ou encore l'azo-isobutyronitrile.

On utilise de 3 à 10% du poids d'agent de réticulation et une quantité d'agent de fonctionnalisation calculée de façon à obtenir 0,2 à 5 mEq de fonction acide carboxylique par gramme de résine sèche (directement ou après saponification); le complément à 100% en poids est constitué par le monomère destiné à constituer la maille.

Dans le cas de la polymérisation en bloc, la solution aqueuse contenant les différents monomères et l'initiateur est soumise à une polymérisation en phase homogène. Le gel transparent qui se forme est rapidement broyé, lavé et tamisé; ce procédé donne un rendement quantitatif, en particulier de copolymères de taille homogène.

La polymérisation en suspension fournit directement la résine sous forme de particules sphériques; celles-ci sont lavées avec beaucoup de soin, séchées. Leur taille est aussi très homogène; le diamètre étant compris entre 0,1 et 0,25 mm (détermination microscopique).

Dans ce cas, à la solution homogène contenant les différents monomères, on ajoute un l'agent initiateur de la polymérisation et l'agent émulsifiant.

L'ensemble est versé dans une phase liquide organique, non miscible à l'eau, maintenue sous agitation. La vitesse d'agitation est réglée de façon à obtenir une émulsion ayant la taille de gouttelettes voulue. La polymérisation est alors initiée, soit par addition de TMEDA, soit par un léger chauffage.

Le mélange est ensuite agité jusqu'à son terme; les perles de résines obtenues sont lavées avec un solvant hydrocarboné, puis à l'eau, à l'éthanol et à l'éther éthylique. La résine est finalement séchée à température ambiante sous vide poussé.

Comme phase organique liquide, utilisable, on peut citer par exemple les huiles végétales (huile de soja, huile d'arachide, huile de tournesol, etc.) ou minérales (huile de paraffine, huile de silicone).

L'agent émulsifiant peut être l'un des produits connus sous la dénomination commerciale Span, Arlacel ou Tween, à la concentration de 0,1 à 4% en volume.

Des exemples ci-après illustrent les différentes techniques de polymérisation ainsi que les différentes résines revendiquées dans la présente invention, sans la limiter.

A) *Copolymérisation en bloc*

*Exemple 1:*

*Copolymérisation d'acrylylpyrrolidine (abrévia-*

*tion AP), de l'éthylène bis acrylamide, encore appelé N,N'-diacrylyldiamino-1,2 éthane (abréviation EBA) et de l'ester méthylique de la N-acrylylglycine (abréviation A Gly O $CH_3$).*

On dissout 0,12 mol (15 g) d'AP, 7,8 · $10^{-3}$ mol (1,31 g) d'EBA et 1,631 · $10^{-2}$ mol (2,33 g) d'ester méthylique de la N-acrylylglycine dans 34,2 ml d'éthanol anhydre. La solution incolore est dégazée 2 min au sonicateur. Par addition de 18 ml d'eau, la solution devient opaque. On laisse dégazer encore 2 min et on ajoute 21,6 ml de tampon phosphate (pH 6,0). Le dégazage est poursuivi jusqu'à retour à une solution limpide. On ajoute alors 3,16 g de persulfate d'ammonium et laisse dissoudre en dégazant. On ajoute alors 0,53 ml de TMEDA (N,N,N',N'-tétraméthyldiamino-1,2 éthane). Il se produit rapidement une élévation de température et la solution se prend en un gel transparent. On abandonne 0,5 h. Après broyage au broyeur Thomas, on laisse décanter dans l'eau afin d'éliminer les fines surnageantes. Cette opération est répétée une deuxième fois. Après filtration, la résine est successivement lavée avec de l'eau, un mélange eau/acétone 50/50 de l'acétone et finalement à l'éther. La poudre blanche obtenue est séchée 12 h à 80° C sous (2 mm) 2,66 mbar. On isole ainsi 13,86 g de résine blanche. Rdt 74,4%.

Pour transformer cette résine ester en résine acide, on place les 13,86 g précédemment obtenus dans 300 ml de soude molaire et on agite pendant 2 h à 25° C. Après filtration la résine est lavée successivement à l'eau, à l'acide chlorhydrique (10%), à l'eau jusqu'à neutralité, à la soude molaire (500 ml), à l'eau jusqu'à neutralité, à l'acide chlorhydrique (10%), et finalement à l'eau jusqu'à neutralité. On sèche comme précédemment à 80° C sous 2 mm de mercure. Après tamisage pour éliminer les particules dont le diamètre est supérieur à 0,25 mm, on obtient 8,8 g de poudre blanche. Le pKa de la forme OH est de 4,4.

*Exemple 2:*

*Copolymérisation AP, EBA, ester méthylique de la N-acrylyl β alanine (abréviation A β Ala O $CH_3$)*

Des conditions identiques de polymérisation sont utilisées: à partir de 15 g (0,12 mol) d'AP, 1,31 g (7,8 · $10^{-3}$ mol) d'EBA et 2,55 g (1,631 · $10^{-2}$ mol) d'ester méthylique de la N-acrylyl β alanine dans 34,2 ml d'éthanol et avec les mêmes quantités d'eau (18 ml), de tampon phosphate (pH 6,0) (21,6 ml), de persulfate d'ammonium (3,16 g) et de TMEDA (0,53 ml), on isole, après saponification dans des conditions identiques à celles décrites dans le paragraphe précédent et après tamisage, 9,43 g de résine sèche et blanche. Le pKa de la forme OH est 4,8.

*Exemple 3:*

*Copolymérisation AP, EBA, ester méthylique de l'acide N-acrylyl ε aminocaproïque (abréviation A ε Cap O $CH_3$)*

Les mêmes conditions de polymérisation que dans les paragraphes précédents sont utilisées à partir des monomères suivants: N-acrylylpyrrolidine (0,12 mol, 15 g), EBA (0,0078 mol, 1,31 g) et N-acrylyl ε aminocaproate de méthyle (16,31 · $10^{-3}$ mol, 3 g). On isole ainsi, après élimination des fines, 13 g (68%) de résine blanche et sèche.

L'hydrolyse dans les mêmes conditions que précédemment conduit, après tamisage, à 10,6 g de résine acide sèche et blanche. Le pKa de la forme OH est 5,1.

*Exemple 4:*

*Copolymérisation AP, EBA, ester méthylique de l'acide N-acrylyl γ amino butyrique (abréviation A γ But O $CH_3$)*

A partir de 15 g (0,12 mol) de AP, 1,31 g (7,8 · $10^{-3}$ mol) de EBA et 2,76 g (0,01631 mol) d'ester méthylique de l'acide N-acrylyl γ amino butyrique, on isole 12 g de résine ester sèche. Après hydrolyse dans 300 ml de soude molaire, on obtient par tamisage 10,8 g de résine acide sèche. Le pKa de la forme OH est 5,0.

*Exemple 5:*

*Copolymérisation AP, EBA, ester méthylique de la N-acrylyl L-leucine (abréviation A(L)Leu O $CH_3$).*

Les quantités de monomères utilisées sont: 15 g de N-acrylylpyrrolidine, 1,31 g de EBA, 3,24 g d'ester méthylique de la N-acrylyl L-leucine.

La résine ester sèche (10,05 g) est hydrolysée par 300 ml de soude molaire. Après traitement comme indiqué précédemment et tamisage, on isole 8,49 g de résine acide. Le pKa de la forme OH est 4,8.

*Exemple 6:*

*Copolymérisation AP, EBA, ester méthylique de la N-acrylyl L-valine (abréviation A(L)Val O $CH_3$)*

On emploie 15 g de N-acrylylpyrrolidine, 1,31 g de EBA et 3 g d'ester méthylique de la N-acrylyl L-valine. La polymérisation et les traitements habituels donnent 9,96 g de résine ester sèche. L'hydrolyse par 300 ml de soude molaire conduit, dans les mêmes conditions que ci-dessus, à 7,64 g de résine acide blanche et sèche. Le pKa de la forme OH est 5,0.

*Exemple 7:*

*Copolymérisation AP, EBA, ester méthylique de la N-acrylyl L-proline (abréviation A(L)Pro O $CH_3$)*

A partir de 15 g (0,12 mol) de N-acrylylpyrrolidine, 1,31 g (0,0078 mol de EBA) et 2,98 g d'ester méthylique de la N-acrylyl L-proline (16,31 · $10^{-3}$ mol), on isole 15,03 g (77,7%) de résine sèche. L'hydrolyse dans 300 ml de soude molaire conduit, après tamisage, à 11 g de résine acide sèche. Le pKa de la forme OH est 4,7.

*Exemple 8:*

*Copolymérisation de l'acrylylmorpholine (abréviation AM), EBA, ester méthylique de l'acide N-acrylyl ε amino caproïque*

5 g (0,0355 mol) de N-acrylylmorpholine, 0,38 g (0,0023 mol) de EBA et 0,995 g (5,38 ·

$10^{-3}$ mol) de N-acrylyl ε amino caproate de méthyle sont dissous dans 10,4 ml d'éthanol. Après dégazage au sonicateur, on ajoute successivement, dans les conditions indiquées dans le premier exemple, 6 ml d'eau, 7,2 ml de solution tampon de phosphate disodique 0,1 M, 1,05 g de persulfate d'ammonium et 0,176 ml de TMEDA.

La polymérisation conduit à 4,93 g (77,4%) de résine sèche et blanche. L'hydrolyse fournit, après tamisage, 2,35 g de résine acide sèche, le pKa de la forme OH est 5,1.

*Exemple 9:*

*Copolymérisation AM, EBA, ester méthylique de la N-acrylyl β alanine*

La polymérisation est effectuée dans les conditions du paragraphe précédent, avec comme monomère 5 g (0,0355 mol) de N-acrylylmorpholine, 0,38 g (0,0023 mol) de EBA et 0,844 g ($5,38 \cdot 10^{-3}$ mol) d'ester méthylique de la N-acrylyl β alanine. On isole ainsi 4,24 g (68,2%) de résine ester sèche, donnant, après hydrolyse et tamisage, 2,18 g de résine acide sèche. Le pKa de la forme OH est 4,8.

*Exemple 10:*

*Copolymérisation du N,N-diméthylacrylamide (abréviation ADM), EBA, ester méthylique de l'acide N-acrylyl ε aminocaproïque*

4,653 g (0,047 mol) de N,N-diméthylacrylamide, 0,51 g (0,003 mol) de EBA et 0,955 g ($5,16 \cdot 10^{-3}$ mol) de N-acrylyl ε aminocaproate de méthyle sont dissous dans 13,4 ml d'alcool éthylique. Après dégazage au sonicateur, on ajoute successivement, dans les conditions déjà indiquées, 7,05 ml d'eau, 8,46 ml de solution tampon de phosphate disodique 0,1 M, 1,237 g de persulfate d'ammonium et 0,2 ml de TMEDA. On obtient 4,64 g (73%) de résine ester blanche, donnant après hydrolyse et tamisage 2,44 g de résine acide. Le pKa de la forme OH est 5,0.

*Exemple 11:*

*Copolymérisation ADM, EBA, ester méthylique de la N-acrylyl β alanine*

Dans les conditions du paragraphe précédent, la polymérisation est effectuée à partir de 4,653 g (0,047 mol) de N-diméthylacrylamide, 0,51 g (0,003 mol) de EBA et 0,81 g ($5,16 \cdot 10^{-3}$ mol) d'ester méthylique de la N-acrylyl β alanine.

Il y a formation de 4,51 g (75,75%) de résine ester sèche conduisant, après hydrolyse et tamisage, à 2,36 g de résine acide, le pKa de la forme OH est 4,7.

*Exemple 12:*

*Copolymérisation du N,N-diéthylacrylamide (abréviation ADE), EBA, ester méthylique de la N-acrylyl β alanine*

La polymérisation est effectuée à partir de 2 g de N-diéthylacrylamide, 0,17 g de EBA et 0,32 g d'ester méthylique de la N-acrylyl β alanine dans 4,5 ml d'éthanol, par additions successives de 2,35 ml d'eau, 2,82 ml de tampon au phosphate disodique 0,1 M, 0,42 g de persulfate d'ammonium et 0,067 ml de TMEDA. Après hydrolyse et tamisage, on isole 1,31 g de résine acide sèche. Le pKa de forme OH est 5,5.

*Exemple 13:*

*Copolymérisation, ADE, EBA, ester méthylique de l'acide N-acrylyl ε amino caproïque*

A partir de 6 g de N-diéthylacrylamide, 0,5 g de EBA et 1,12 g de N-acrylyl ε amino caproate de méthyle dans 18,4 ml d'alcool, et par additions successives de 7,05 ml d'eau, 8,46 ml de tampon au phosphate disodique 0,1 M, 1,24 g de persulfate d'ammonium et 0,2 ml de TMEDA, on obtient, après hydrolyse et tamisage, 4,77 g de résine sèche. Le pKa de la forme OH est 5,42.

*Exemple 14:*

*Copolymérisation de N-acrylyl hexaméthylène-imine, EBA, ester méthylique de N-acrylyl β alanine*

A partir de 6 g de N-acrylyl hexaméthylène-imine, 0,33 g de EBA, 0,65 g d'ester méthylique de la N-acrylyl β alanine dans 8,4 ml d'alcool, on ajoute successivement dans les conditions habituelles 5,6 ml d'eau, 6,9 ml de tampon, 1,0 g de persulfate d'ammonium et 0,18 ml de TMEDA.

*Exemple 15:*

*Copolymérisation de la N-acrylyl N'-méthyl-pipérazine, EBA, ester méthylique de la N-acrylyl β alanine*

3,0 g de chlorhydrate de N-acrylyl N'-méthyl-pipérazine, 0,16 g de EBA et 0,3 g d'ester méthylique de la N-acrylyl β alanine dans 4,2 ml d'éthanol sont traités successivement par 2,8 ml d'eau, 3,5 ml de tampon, 0,5 g de persulfate d'ammonium et 0,09 ml de TMEDA.

Dans tous ces exemples, il a été vérifié que les esters obtenus ne s'hydrolysent pas au cours de la polymérisation, les résines ne contiennent aucun $CO_2H$ libre.

*Exemple 16:*

*Copolymérisation AP, méthylènebisacrylamide (MEBA), N-acrylyl β alanine*

On dissout 25 g (0,2 mol) de AP, 2,15 g ($1,3 \cdot 10^{-2}$ mol) de méthylènebisacrylamide et 3,5 g ($2,4 \cdot 10^{-2}$ mol) de N-acrylyl β alanine dans 57 ml d'éthanol absolu. On ajoute 30 ml d'eau puis 36 ml de tampon phosphate (pH 6,0). La solution limpide est dégazée au sonicateur 2 min. Ajouter 5,27 g de persulfate d'ammonium puis 0,88 ml de TMEDA, laisser polymériser 30 min. Le gel transparent est broyé dans un appareil Thomas, lavé à l'eau, à l'éthanol, puis à l'éther. La résine est séchée sous vide. Rdt: 27,6 g.

La résine est alors tamisée. On obtient la répartition en taille suivante:

| | |
|---|---|
| d > 0,25 mm | 4,08 g |
| 0,2 > d > 0,18 mm | 15,18 g |
| 0,18 > d > 0,16 mm | 5,37 g |
| 0,16 > d > 0,125 mm | 0,72 g |

B) *Copolymérisation en suspension*

*Exemple 17:*

*Copolymérisation de la N-méthacrylylmorpholine, EBA, ester méthylique de la N-acrylyl β alanine*

Un mélange de 18,6 g (0,12 mol) de N-méthacrylylmorpholine, de 1,31 g de EBA ($7,8 \cdot 10^{-3}$ mol), de 2,55 g ($1,63 \cdot 10^{-2}$ mol) d'ester méthylique de la N-acrylyl β alanine et de 100 mg d'azo-isobutyronitrile sont ajoutés, sous azote, à une solution agitée d'alcool polyvinylique (3 g) et d'eau (250 ml). Le mélange est chauffé 3 h à 70° C. Le polymère est lavé à l'eau chaude, à l'éthanol, à l'acétone, à l'éthanol et à l'éther. Après séchage sous vide (2 mm de mercure) à 80° C, on isole 14,8 g de billes dont le diamètre est compris entre 0,1 et 0,2 mm.

*Exemple 18:*

*Copolymérisation de la N-méthacrylylmorpholine, EBA, ester méthylique de l'acide N-acrylyl ε aminocaproïque*

A partir d'un mélange de 18,6 g (0,12 mol) de N-méthacrylylmorpholine, de 1,31 g ($7,8 \cdot 10^{-3}$ mol) d'EBA, 3,24 g ($1,631 \cdot 10^{-2}$ mol) d'ester méthylique de l'acide N-acrylyl ε aminocaproïque et de 100 mg d'azo-isobutyronitrile, traité dans les mêmes conditions que précédemment, on isole 12,6 g de polymère.

*Exemple 19:*

*Copolymérisation de AP, EBA, ester méthylique de la N-acrylyl β alanine*

4 g ($3,2 \cdot 10^{-2}$ mol) d'AP, 8 g ($5\ 10^{-2}$ mol) de N-acrylyl β alanine méthyl ester et 1,2 g ($7,14 \cdot 10^{-3}$ mol) d'EBA sont dissous dans 50 ml de tampon phosphate ($KH_2PO_4$ 0,1M ramené à pH 6,0 avec de la soude 1N) et 50 ml d'éthanol. A la solution limpide obtenue, on ajoute 0,6 g de persulfate d'ammonium et 0,3 ml de Span 85. Le mélange est placé dans 400 ml d'huile de paraffine, puis agité jusqu'à obtention de gouttelettes de dimension désirée. Toujours sous agitation, le milieu est désoxygéné par un bullage à l'azote de 20 min. On ajoute alors 0,92 ml de TMEDA. Après 30 min d'agitation, on dilue à l'éther de pétrole, on filtre, lave à l'éther de pétrole, à l'eau, avec un mélange eau/acétone (50/50), à l'acétone, puis à l'éther.

On obtient ainsi des billes de polymère parfaitement sphériques dont on peut faire varier la taille, en ajustant la vitesse de rotation, entre 0,05 et 0,5 mm. Le rendement est voisin de 90%.

La réaction peut s'effectuer également dans l'eau à la place du tampon phosphate.

Les sels de sodium des résines acides sont facilement obtenus en lavant les polymères par des solutions de soude 0,5M. On utilise 30 ml de solution de soude par millilitre de résine acide gonflée. L'excès de soude est éliminé par lavage à l'eau jusqu'à neutralité.

Les taux de gonflement des polymères ont été mesurés dans l'eau et dans différents solvants organiques. Ces mesures ont été réalisées sur des résines broyées. (Tableaux 1 et 2.)

Les polyacrylamides préparés ont été testés comme échangeur de cations en chromatographie d'échange de cations sur trois substrats:

- deux mélanges peptidiques,
- un mélange de protéines basiques (histones provenant de thymus de veau).

Les mélanges peptidiques sont composés de 2 peptides de même séquence qui ne diffèrent uniquement que par la charge:

- Ac Gly Lys Ala Leu Arg Val O Me — 2 charges $> 0$
- Ac GLy Lys Ala Leu Arg Val OH — 1 charge $> 0$
- Ac Gly Lys Leu Arg Val O Me — 2 charges $> 0$
- Ac Gly Lys Leu Arg Val OH — 1 charge $> 0$

Les séparations ont été réalisées dans un gradient de chlorure de sodium à pH constant (pH 5,0, acétate de sodium $10^{-2}$ M), en utilisant des résines tamisées correspondant aux copolymères suivants:

- AP + MEBA + A β Ala O Na
- AP + EBA + A ε Cap O Na
- AP + EBA + A(L)Pro O Na
- AP + EBA + A Gly O Na

La taille des particules est comprise entre 0,1 et 0,25 mm. La charge en groupement carboxylique est voisine de 0,8 mEq/g de résine sèche. Les chromatogrammes, fig. 1 et 2 ci-après, montrent une très bonne séparation entre les composantes des mélanges analysés.

Les histones de thymus de veau ont été également chromatographiées dans un gradient de chlorure de sodium (0 à 2M) (fig. 3). On observe une résolution des différentes composantes, avec un temps d'analyse très court (5 h), un débit rapide (80 ml/h), et un tampon (NaCl) mieux adapté à l'analyse par UV.

Pour une résolution semblable, les chromatographies par échange d'ions d'histones sont effectuées dans des gradients aux formes élaborées de chlorure de guanidinium avec des débits très lents et des temps d'analyse qui peuvent varier entre 12 h et plusieurs jours (J. Bonner et coll., «Method in Enzymology», vol. 126; Grossmann L. et Maldave K. Editeurs, Academic Press, New York, 1968).

Les résines selon l'invention sont donc compatibles avec les protéines et permettent de réaliser facilement de bonnes séparations. Il est à noter de plus que les supports décrits ici admettent des débits très élevés. Il est possible de travailler jusqu'à 300 ml/h avec une colonne 1 × 25 cm.

Sur ces quatre figures, $\lambda = 210$ mm, la solution d'élution est 0,2M en NaCl, les numéros indiqués en abscisses sont ceux de fractions recueillies et les chiffres en ordonnées représentent la densité optique pleine échelle.

*Tableau 1*

*Volume de 1 g de résine en ml*

| Résine / Milieu | AP+EBA | | AP+EBA | | AP+EBA | | AP+EBA | | AP+EBA | | AP+EBA | | AP+EBA | |
|---|---|---|---|---|---|---|---|---|---|---|---|---|---|---|
| | A Gly OH * | A Gly ONa ** | A β Ala OH | A β Ala ONa | A ε Cap OH | A ε Cap ONa | A Val OH | A Val ONa | A Leu OH | A Leu ONa | A Pro OH | A Pro ONa | A Ala OH | A Ala ONa |
| Sèche | 1,9 | 2,2 | 2,2 | 2,1 | 2,2 | 1,6 | 1,8 | 1,5 | 1,9 | 2,8 | 3,0 | 2,4 | 2,8 | 2,0 |
| Eau | 11,7 (×6)*** | 22 (×10)*** | 11,0 (×5) | 26,2 (×12,5) | 8,8 (×4) | 10,5 (×10,5) | 9,2 (×5) | 12,0 (×2) | 7,8 (×4) | 23,8 (×2,5) | 18,0 (×6) | 21,4 (×9) | 28 (×10) | 26 (×13) |
| $CH_3OH$ | 10,3 (×5,4) | 19,8 (×9) | 12,1 (×5,5) | 20,0 (×9,5) | 8,8 (×4) | 10,5 (×10,5) | 9,4 (×5,2) | 12,0 (×8) | 11,6 (×6) | 22,4 (×8) | 15,0 (×5) | 16,8 (×7) | 22,4 (×8) | 16 (×8) |
| $C_2H_5OH$ | 12,0 (×6,3) | 12,1 (×5,5) | 14,3 (×6,5) | 18,9 (×9) | 12,1 (×5,5) | 9,5 (×9,5) | 13,0 (×7,2) | 13,5 (×9) | 10,7 (×5,5) | 22,4 (×8) | 18,0 (×6) | 19,2 (×8) | 22,4 (×8) | 14 (×7) |
| $CH_2Cl_2$ | 13,3 (×7,0) | | 11,0 (×5) | | 11 (×5) | 9,0 (×9) | 13,5 (×7,5) | | 11,6 (×6) | | 15,0 (×5) | | 22,4 (×8) | |
| $CHCl_3$ | 14,2 (×7,5) | | 12,1 (×5,5) | | 12,1 (5,5) | 6,0 (×6) | 11,7 (×6,5) | | 11,6 (×6) | | 12,0 (×4) | | 19,6 (×7) | |
| $C_5H_5N$ | 15,2 (×8,0) | | 18,7 (×8,5) | | 11 (×5) | | 19,8 (×11) | | 19,4 (×10) | | 30,0 (×10) | | 16,8 (×6) | |
| DMF | 10,8 (×5,7) | | 9,9 (×4,5) | | 8,8 (×4) | | 8,5 (×4,7) | | 7,8 (×4) | | 12,0 (×4) | | 14 (×5) | |

* L'abréviation A Gly OH correspond à la N-acrylylglycine. Il en est de même pour tous les autres acides aminés dont l'abréviation contient la particule «OH».

** L'abréviation A Gly O Na correspond au sel de sodium de la N-acrylylglycine. Il en est de même pour tous les autres dérivés d'aminoacides dont l'abréviation contient la particule «ONa».

*** Nombre par lequel le volume spécifique de la résine sèche est multiplié.

*Tableau 2*

*Volume de 1 g de résine en ml*

| Milieu \ Résine | ADM+EBA | | ADM+EBA | | AM+EBA | | AM+EBA | | ADE+EBA | | ADE+EBA | |
|---|---|---|---|---|---|---|---|---|---|---|---|---|
| | A ε Cap OH | A ε Cap ONa | A β Ala OH | A β Ala ONa | A ε Cap OH | A ε Cap ONa | A β Ala OH | A β Ala ONa | A ε Cap OH | A ε Cap ONa | A β Ala OH | A β Ala ONa |
| Sèche | 3,1 | 3,5 | 2,9 | 4,1 | 4,5 | 7,1 | 2,6 | 3,2 | 4,2 | 6,6 | 3,0 | 4,2 |
| Eau | 15,5 (×5)* | 56 (×16) | 20,3 (×7) | 65,6 (×16) | 18 (×4) | 78 (×11) | 20,8 (×8) | 51,2 (×16) | 16,8 (×4) | 46,2 (×7) | 24 (×8) | 54,6 (×13) |
| $CH_3OH$ | 24,8 (×3) | 26,25 (×7,5) | 23,2 (×8) | 32,8 (×8) | 13,5 (×3) | 56,8 (×8) | 13 (×5) | 17,6 (×5,5) | 21 (×5) | 26,4 (×4) | 24 (×8) | 42 (×10) |
| $C_2H_5OH$ | 21,7 (×7) | 21 (×6) | 26,1 (×9) | 28,7 (×7) | 13,5 (×3) | 35,5 (×5) | 7,8 (×3) | 8,0 (×2,5) | 21 (×5) | 11,2 (×3) | 30 (×10) | 25,2 (×6) |
| $CH_2Cl_2$ | 27,9 (×9) | | 29 (×10) | | 27 (×6) | | 20,8 (×8) | | 50,4 (×12) | | 33 (×11) | |
| $CH\ Cl_3$ | 24,8 (×8) | | 26,1 (×9) | | 27 (×6) | | 26 (×10) | | 46,2 (×11) | | 30 (×10) | |
| $C_5H_5N$ | 31 (×10) | | 26,1 (×9) | | 27 (×6) | | 28,6 (×11) | | 16,8 (×4) | | 30 (×10) | |
| DMF | 27,9 (×9) | | 14,5 (×5) | | 40,5 (×9) | | 31,2 (×12) | | 25,2 (×6) | | 15 (×5) | |

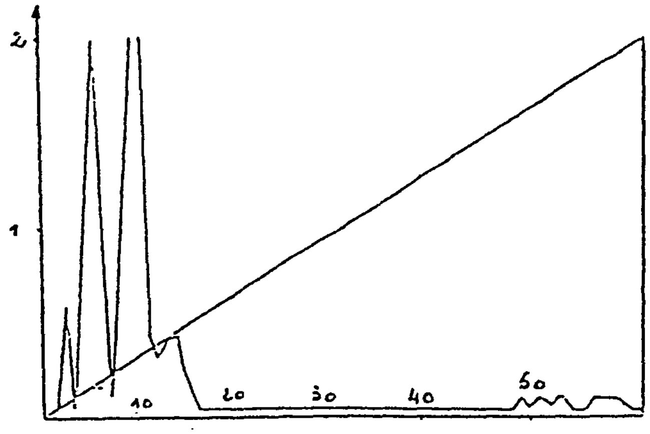


chromatographie de:
Ac Gly Lys Ala Leu Arg Val OMI + Ac Gly Lys Ala Leu Arg Val OH sur colonne d'acrylyl-pyrrolidine, de méthylènebisacrylamide, d'acrylyl β alanine
débit 89 ml/h
fractions 15 mm

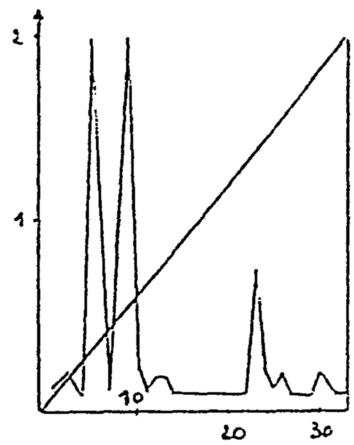


chromatographie de:
Ac Gly Lys Leu Arg Val OMI + Ac Gly Lys Leu Arg Val OH sur une colonne d'acrylylpyrrolidine, d'éthylènebisacrylamide et d'acrylyl β alanine
débit 80 ml/h
fractions 15 mm

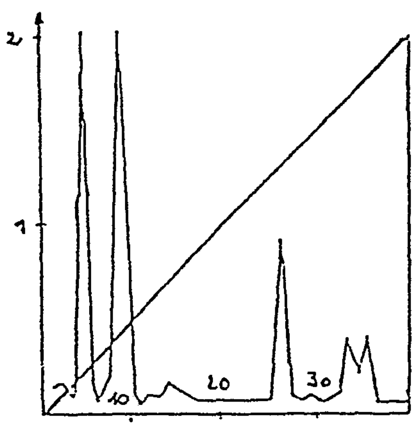


chromatographie de:
Ac Gly Lys Leu Arg Val OMI + Ac Gly Lys Leu Arg Val OH sur une colonne d'acrylylpyrrolidine, de méthylènebisacrylamide et d'acrylyl ε aminocaproïque
débit 80 ml/h
fractions 15 mm

chromatographie de:
Ac Gly Lys Leu Arg Val OMI + Ac Gly Lys Leu Arg Val OH sur une colonne d'acrylylpyrrolidine, d'éthylènebisacrylamide et d'acrylylproline
débit 50 ml/h
fractions 15 mm

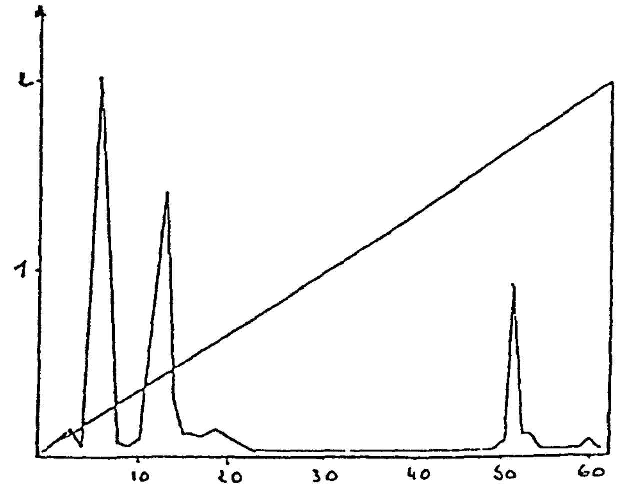


FIGURE 1

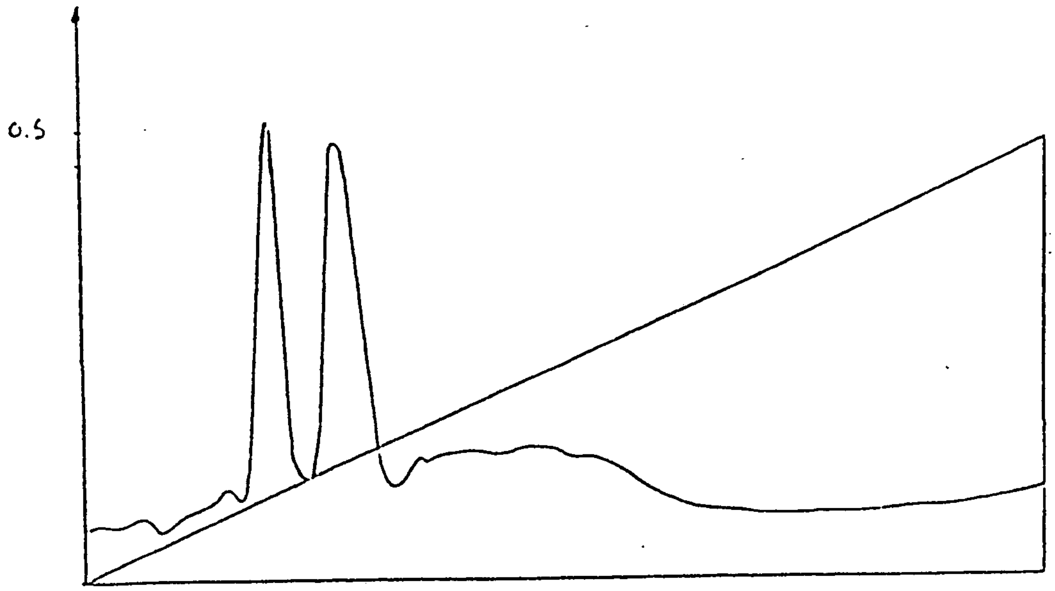


Fig. 2: chromatographie de: Ac Gly Lys Leu Arg Val OMI + Ac Gly Lys Leu Arg Val OH sur une colonne d'acrylylpyrrolidine, d'éthylènebisacrylamide et d'acrylylglycine
débit 80 ml/h – durée 8 h – λ = 220 mm.

En abscisses sont portées les fractions et en ordonnées la densité optique pleine échelle. La solution d'élution est 0,2M en NaCl.

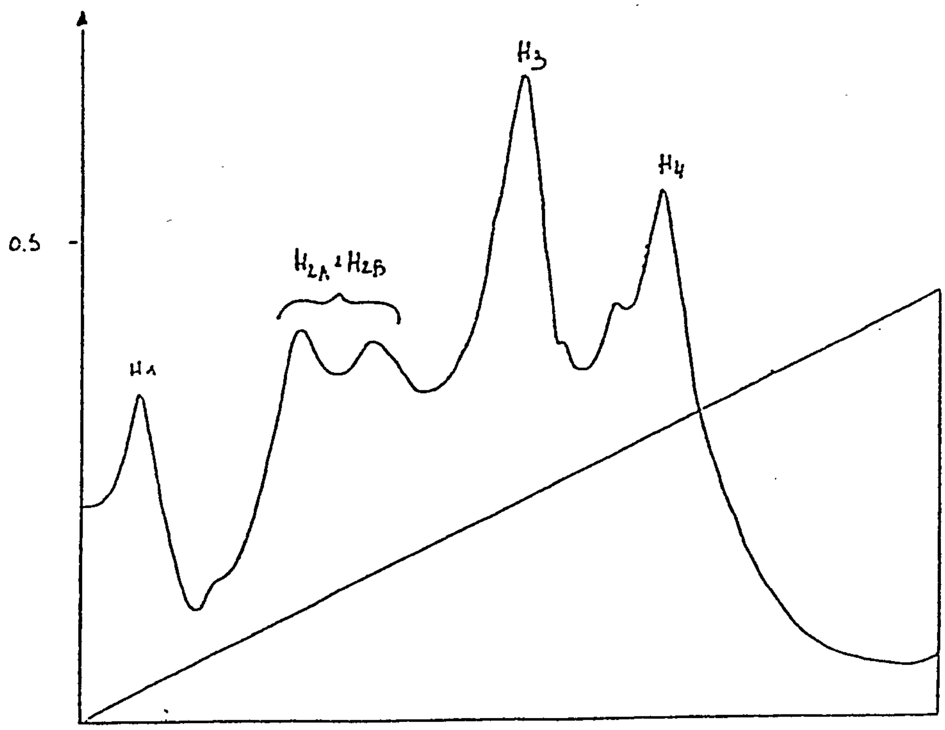


Fig. 3: chromatographie d'un mélange d'histones sur une colonne d'acrylylpyrrolidine, d'éthylènebisacrylamide et d'acrylyl β alanine
débit 80 ml/h – durée 5 h – λ = 210 mm.

En abscisses sont portées les fractions et en ordonnées la densité optique pleine échelle. La solution d'élution est 2M en NaCl.

**Revendications** pour les Etats contractants BE, CH, DE, FR, GB, IT, LI, LU, NL, SE

1. Copolymères acryliques, caractérisés en ce qu'ils se composent:
– de 30 à 90%, en poids, d'un premier monomère (I) défini comme étant la maille du support et qui consiste en une N-acrylylpolyméthylèneimine de formule:

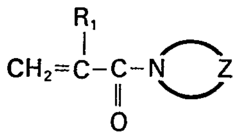 (1)

avec $R_1$ = H ou $-CH_3$

$Z = -(CH_2)_{n1}-$ avec n = 4, 5 ou 6

ou

$-(CH_2)_2-X-(CH_2)_2-$

avec X = O ou $N-CH_3$

ou un N-acrylyldialcoylamide, de formule:

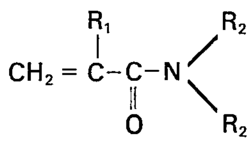 (2)

avec $R_1$ ayant la signification précédente

$R_2 = -CH_3$ ou $-C_2H_5$.

– de 2 à 50%, en poids, d'un second monomère (II) défini comme étant l'agent de réticulation et qui consiste en un N,N'-bisacrylyldiaminoalcane de formule:

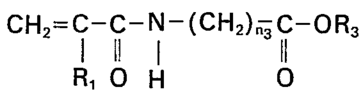

avec $n_2$ = 1 ou 2.

– de 2 à 65%, en poids, d'un troisième monomère (III) défini comme étant l'agent de fonctionnalisation et qui consiste en un N-acrylylaminoacide ou ester, de formule:

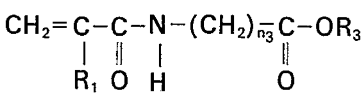

avec
$R_1$ = H ou $-CH_3$
$R_3$ = H ou $-CH_3$
$n_3$ = 1, 2, 3 ou 5
ou un N-acrylylaminoacide (ou ester) asymétrique de série L, de formule:

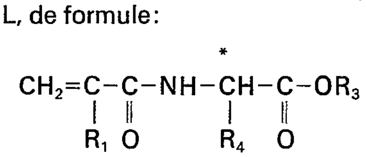

avec $R_1$ = H ou $-CH_3$
$R_3$ ayant la signification précédente
$R_4 = -CH_3$
$-CH(CH_3)_2$
$-CH_2-CH(CH_3)_2$

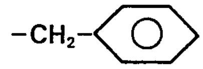

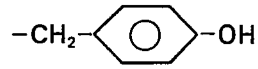

$-CH_2-CH_2-S-CH_3$
$-(CH_2)_4-NH_2$

ou encore la N-acrylyl (L) proline, ou son ester méthylique, de formule:

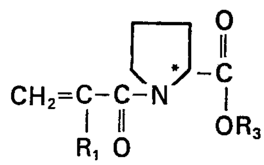

avec $R_1$=H ou $-CH_3$

$R_3$ ayant la signification précédente.

2. Copolymères selon la revendication 1, caractérisé en ce qu'ils se composent de 70 à 90% d'un monomère (I), de 3 à 10% d'un monomère (II) et de 10 à 20% d'un monomère (III).

3. Procédé de préparation de copolymères selon la revendication 1, caractérisé en ce qu'il consiste à effectuer la polymérisation radicalaire des monomères (I), (II) et (III).

4. Procédé selon la revendication 3, caractérisé en ce que la polymérisation est effectuée en solution aqueuse, à une température de 0 à 50°C, en présence d'un initiateur de polymérisation radicalaire.

5. Procédé selon la revendication 4, caractérisé en ce que la solution aqueuse peut être polymérisée en bloc.

6. Procédé selon la revendication 3, caractérisé en ce que, dans le cas d'une polymérisation en émulsion, la solution aqueuse est sous la forme d'une émulsion au sein d'une phase liquide organique, non miscible à l'eau.

7. Utilisation d'un copolymère selon l'une des revendications 1 ou 2 comme résines échangeuses de cations.

**Revendications** pour l'Etat contractant: AT

1. Procédé de préparation de copolymères acryliques se composant:
– de 30 à 90%, en poids, d'un premier monomère (I) défini comme étant la maille du support et qui consiste en une N-acrylylpolyméthylèneimine de formule:

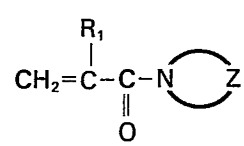

avec $R_1$ = H ou $-CH_3$

$Z = -(CH_2)_{n1}-$ avec $n_1$ = 4, 5 ou 6

ou

$-(CH_2)_2-X-(CH_2)_2-$

avec X = O ou $N-CH_3$

ou un N-acrylyldialcoylamide, de formule:

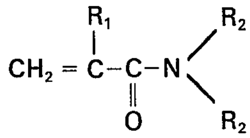

avec $R_1$ ayant la signification précédente

$R_2 = -CH_3$ ou $-C_2H_5$

– de 2 à 50%, en poids, d'un second monomère (II) défini comme étant l'agent de réticulation et qui consite en un N,N'-bisacrylyldiaminoalcane de formule:

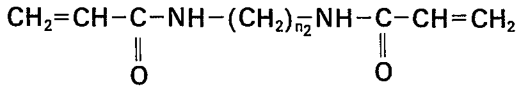

avec $n_2 = 1$ ou 2.

– de 2 à 65%, en poids, d'un troisième monomère (III) défini comme étant l'agent de fonctionnalisation et qui consiste en un N-acrylylaminoacide ou ester, de formule:

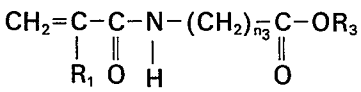

avec
$R_1 = H$ ou $-CH_3$
$R_3 = H$ ou $-CH_3$
$n_3 = 1, 2, 3$ ou 5

ou un N-acrylylaminoacide (ou ester) asymétrique de série L, de formule:

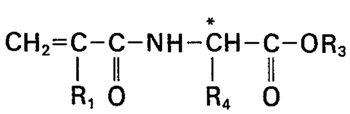

avec $R_1 = H$ ou $-CH_3$
$R_3$ ayant la signification précédente
$R_4 = -CH_3$

$-CH(CH_3)_2$

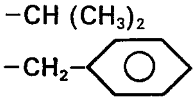

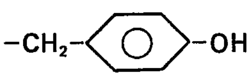

$-CH_2-CH_2-S-CH_3$

$-(CH_2)_4-NH_2$

ou encore la N-acrylyl (L) proline, ou son ester méthylique, de formule:

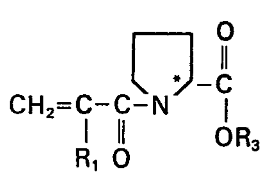

avec $R_1 = H$ ou $-CH_3$

$R_3$ ayant la signification précédente, caractérisé en ce qu'il consiste à effectuer la polymérisation radicalaire des monomères (I), (II) et (III).

2. Procédé selon la revendication 1, caractérisé en ce que la polymérisation est effectuée en solution aqueuse, à une température de 0 à 50° C, en présence d'un initiateur de polymérisation radicalaire.

3. Procédé selon la revendication 2, caractérisé en ce que la solution aqueuse peut être polymérisée en bloc.

4. Procédé selon la revendication 1, caractérisé en ce que, dans le cas d'une polymérisation en émulsion, la solution aqueuse est sous la forme d'une émulsion au sein d'une phase liquide organique, non miscible à l'eau.

**Patentansprüche** für die Vertragsstaaten BE, CH, DE, FR, GB, IT, LI, LU, NL, SE

1. Acrylpolymere, dadurch gekennzeichnet, dass sie: zu 30 bis 90 Gew.-% aus einem ersten Monomeren (I), welches als Stützmasche definiert ist und welches entweder aus einem N-Acrylylpolymethylenimin der Formel:

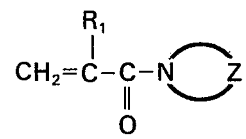

wobei $R_1 = H$ oder $-CH_3$

$Z = -(CH_2)_{n_1}-$ wobei $n_1 = 4, 5$ oder 6

oder

$-(CH_2)_2-X-(CH_2)_2-$

mit X = O oder $N-CH_3$ ist,

oder aus einem N-Acrylyldialcoylamid der Formel:

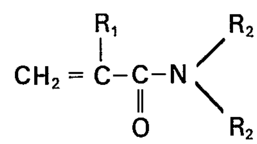

wobei $R_1$ die oben genannte Bedeutung hat und

$R_2 = -CH_3$ oder $-C_2H_5$ ;

zu 2 bis 50 Gew.-% aus einem zweiten Monomeren (II), das als Vernetzungsmittel definiert ist und aus einem N,N'-Bisacrylyldiaminoalkan der Formel:

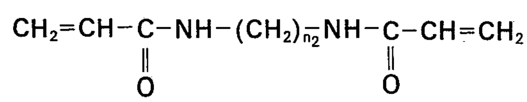

mit $n_2 = 1$ oder 2, sowie zu

2 bis 65 Gew.-% aus einem dritten Monomeren (III), welches als funktionelles Mittel definiert ist und entweder aus einer N-Acrylylaminosäure oder einem Ester der Formel:

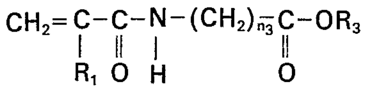

mit
$R_1$ = H ou $-CH_3$
$R_3$ = H ou $-CH_3$
$n_3$ = 1, 2, 3 ou 5
oder aus einer asymmetrischen, L-Form aufweisenden N-Acrylylaminosäure (oder einem Ester) der Formel:

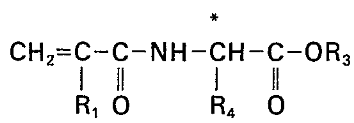

mit
$R_1$ = H oder $-CH_3$
$R_3$ mit obiger Bedeutung und
$R_4$ = $-CH_3$
$-CH(CH_3)_2$
$-CH_2-CH(CH_3)_2$

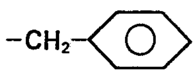

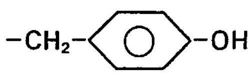

$-CH_2-CH_2-S-CH_3$
$-(CH_2)_4-NH_2$
oder aus dem N-Acrylyl(L)prolin oder dessen Methylester der Formel:

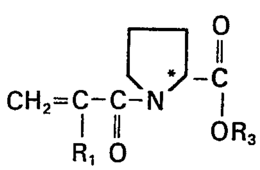

mit
$R_1$ = H ou $-CH_3$ und
$R_3$ mit der oben angegebenen Bedeutung bestehen.

2. Copolymere nach Anspruch 1, dadurch gekennzeichnet, dass sie zu 70 bis 90% aus einem Monomeren (I), zu 3 bis 10% aus einem Monomeren (II) und zu 10 bis 20% aus einem Monomeren (III) bestehen.

3. Verfahren zur Herstellung des Copolymeren gemäss dem Anspruch 1, dadurch gekennzeichnet, dass man die Radikalpolymerisation der Monomeren (I), (II) und (III) durchführt.

4. Verfahren nach Anspruch 3, dadurch gekennzeichnet, dass die Polymerisation in wässeriger Lösung bei einer Temperatur zwischen 0 und 50° C in Gegenwart eines die Radikalpolymerisation auslösenden Initiators ausführt.

5. Verfahren nach Anspruch 4, dadurch gekennzeichnet, dass die wässerige Lösung im Ganzen polymerisiert werden kann.

6. Verfahren nach Anspruch 3, dadurch gekennzeichnet, dass im Falle der Polymerisation in der Emulsion die wässerige Lösung in einer mit ihr nicht mischbaren organischen Flüssigkeit emulgiert ist.

7. Verwendung eines Copolymeren gemäss einem der Ansprüche 1 oder 2 als Kationenaustauschharz.

**Patentansprüche** für den Vertragsstaat: AT

1. Verfahren zur Herstellung von Acrylcopolymeren, die zu 30 bis 90 Gew.-% aus einem ersten Monomeren (I), welches als Stützmasche definiert ist und welches entweder aus einem N-Acrylylpolymethylenimin der Formel:

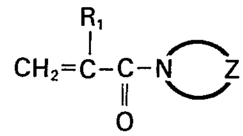

wobei $R_1$ = H oder $-CH_3$

Z = $-(CH_2)_{n_1}-$ wobei $n_1$ = 4, 5 oder 6

oder

$-(CH_2)_2-X-(CH_2)_2-$

mit X = O oder N—$CH_3$ ist,

oder aus einem N-Acrylyldialcoylamid der Formel:

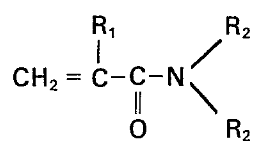

wobei $R_1$ die oben genannte Bedeutung hat und

$R_2$ = $-CH_3$ oder $-C_2H_5$;

zu 2 bis 50 Gew.-% aus einem zweiten Monomeren (II), das als Vernetzungsmittel definiert ist und aus einem N,N'-Bisacrylyldiaminoalkan der Formel:

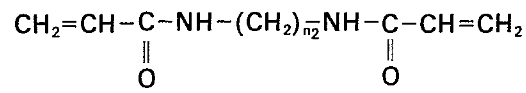

mit $n_2$ = 1 oder 2, sowie zu

2 bis 65 Gew.-% aus einem dritten Monomeren (III), welches als funktionelles Mittel definiert ist und entweder aus einer N-Acrylylaminosäure oder einem Ester der Formel:

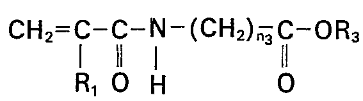

mit
$R_1$ = H ou $-CH_3$
$R_3$ = H ou $-CH_3$
$n_3$ = 1, 2, 3 ou 5
oder aus einer asymmetrischen, L-Form aufweisenden N-Acrylylaminosäure (oder einem Ester) der Formel:

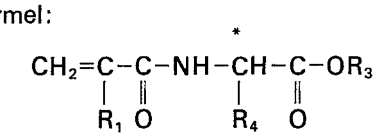

mit

$R_1$ = H oder $-CH_3$

$R_3$ mit obiger Bedeutung und

$R_4 = -CH_3$

$-CH(CH_3)_2$

$-CH_2-CH(CH_3)_2$

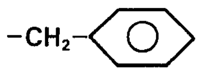

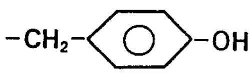

$-CH_2-CH_2-S-CH_3$

$-(CH_2)_4-NH_2$

oder aus dem N-Acrylyl(L)prolin oder dessen Methylester der Formel:

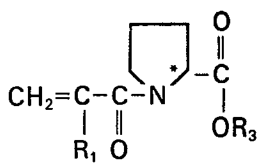

mit

$R_1$ = H ou $-CH_3$ und

$R_3$ mit der oben angegebenen Bedeutung bestehen, dadurch gekennzeichnet, dass man die Radikalpolymerisation der Monomeren (I), (II) und (III) durchführt.

2. Verfahren nach Anspruch 1, dadurch gekennzeichnet, dass die Polymerisation in wässeriger Lösung bei einer Temperatur zwischen 0 und 50° C in Gegenwart eines die Radikalpolymerisation auslösenden Initiators ausführt.

3. Verfahren nach Anspruch 2, dadurch gekennzeichnet, dass die wässerige Lösung im Ganzen polymerisiert werden kann.

4. Verfahren nach Anspruch 1, dadurch gekennzeichnet, dass im Falle der Polymerisation in der Emulsion die wässerige Lösung in einer mit ihr nicht mischbaren organischen Flüssigkeit emulgiert ist.

**Claims** for the Contracting States BE, CH, DE, FR, GB, IT, LI, LU, NL, SE

1. Acrylic copolymers characterised in that they comprise:

from 30 to 90% by weight of a first monomer (I) defined as being the matrix and which consists of an N-acrylylpolymethyleneimine of the formula:

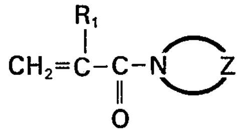

with $R_1$ = H or $-CH_3$

$Z = -(CH_2)_{n_1}-$ with $n_1$ = 4, 5 or 6

or

$-(CH_2)_2-X-(CH_2)_2-$

with X = O or N—$CH_3$

or an N-acrylyldialcoylamide of formula:

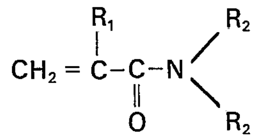

with $R_1$ having the above meaning

$R_2 = -CH_3$ or $-C_2H_5$

from 2 to 50% by weight of a second monomer (II) defined as being the cross-linking agent and which consists of a N,N'-bisacrylyldiaminoalkane of the formula:

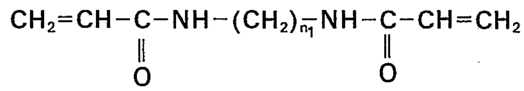

with $n_2$ = 1 or 2

from 2 to 65% by weight of a third monomer (III) defined as being the activation agent and which consists of an N-acrylylaminoacid or ester of formula:

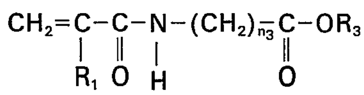

with

$R_1$ = H or $-CH_3$

$R_3$ = H or $-CH_3$

$n_3$ = 1, 2, 3 or 5

or an asymmetric N-acrylylaminoacid (or ester) of the L series of formula:

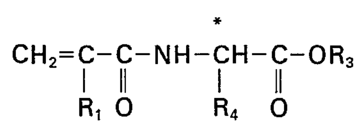

with

$R_1$ = H or $-CH_3$

$R_3$ having the above meaning

$R_4 = -CH_3$

$-CH(CH_3)_2$

$-CH_2-CH(CH_3)_2$

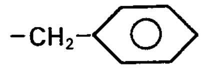

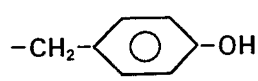

$-CH_2-CH_2-S-CH_3$

$-(CH_2)_4-NH_2$

or again N-acrylyl (L) proline, or its methyl ester, of formula:

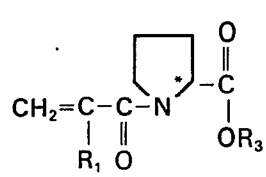

with
$R_1 = H$ or $-CH_3$
$R_3$ having the above meaning.

2. Copolymers according to Claim 1, characterised in that they comprise from 70 to 90% of a monomer (I), from 3 to 10% of a monomer (II) and from 10 to 20% of a monomer (III).

3. Process of preparation of copolymers according to Claim 1, characterised in that it consists of forming the radical polymerisation of the monomers (I), (II) and (III).

4. Process according to Claim 3, characterised in that the polymerisation is performed in aqueous solution, in a temperature from 0 to 50° C, in the presence of an initiator for radical polymerisation.

5. Process according to Claim 4, characterised in that the aqueous solution can be block-polymerised.

6. Process according to Claim 3, characterised in that, in the case of an emulsion polymerisation, the aqueous solution is in the form of an emulsion in a liquid organic phase, not miscible with water.

7. Use of a copolymer according to one of Claims 1 or 2 as cation exchange resins.

**Claims** for the Contracting State: AT

1. Process of preparing acrylic copolymers comprising from 30 to 90% by weight of a first monomer (I) defined as being the support matrix and which consists of an N-acrylylpolymethyleneimine of the formula:

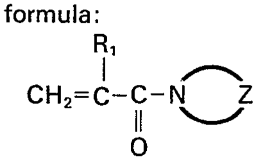

with $R_1 = H$ or $-CH_3$

$Z = -(CH_2)_{n_1}-$ with $n_1 = 4, 5$ or $6$

or

$-(CH_2)_2-X-(CH_2)_2-$

with X = O or $N-CH_3$

or an N-acrylyldialcoylamide of formula:

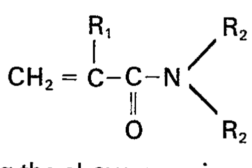

with $R_1$ having the above meaning
$R_2 = -CH_3$ or $-C_2H_5$

from 2 to 50% by weight of a second monomer (II) defined as being the cross-linking agent and which consists of a N,N'-bisacrylyldiaminoalkane of the formula:

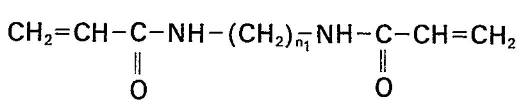

with $n_2 = 1$ or $2$

from 2 to 65% by weight of a third monomer (III) defined as being the activation agent and which consists of an N-acrylylaminoacid or ester of formula:

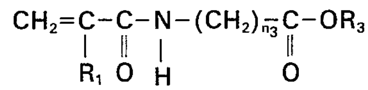

with
$R_1 = H$ or $-CH_3$
$R_3 = H$ or $-CH_3$
$n_3 = 1, 2, 3$ or $5$

or an asymmetric N-acrylylaminoacid (or ester) of the L series of formula:

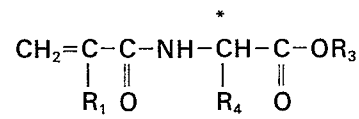

with
$R_1 = H$ or $-CH_3$
$R_3$ having the above meaning
$R_4 = -CH_3$
$-CH(CH_3)_2$
$-CH_2-CH(CH_3)_2$

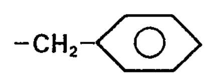

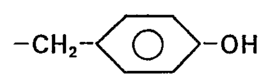

$-CH_2-CH_2-S-CH_3$
$-(CH_2)_4-NH_2$

or again N-acrylyl (L) proline, or its methyl ester, of formula:

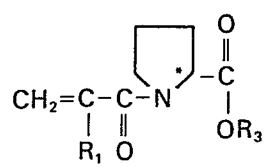

with
$R_1 = H$ or $-CH_3$
$R_3$ having the above meaning, characterised in that it consists of forming the radical polymerisation of the monomers (I), (II) and (III).

2. Process according to Claim 1, characterised in that the polymerisation is performed in aqueous solution, at a temperature from 0 to 50° C, in the presence of an initiator for radical polymerisation.

3. Process according to Claim 2, characterised in that the aqueous solution can be block-polymerised.

4. Process according to Claim 1, characterised in that, in the case of an emulsion polymerisation, the aqueous solution is in the form of an emulsion in an organic liquid phase, not miscible with water.